# EUROPÄISCHE PATENTSCHRIFT

(11) **EP 2 265 232 B1**
(45) Veröffentlichungstag und Bekanntmachung des Hinweises auf die Patenterteilung: **11.01.2012**
(21) Anmeldenummer: 08733792.9
(22) Anmeldetag: 12.04.2008
(51) Int. Cl.: A61F 9/007

(54) **CHIRURGISCHES HILFSMITTEL FÜR DIE OPHTHALMOLOGIE**
SURGICAL AID FOR OPHTHALMOLOGY
AIDE CHIRURGICALE À USAGE OPHTALMOLOGIQUE

(43) Veröffentlichungstag der Anmeldung: 29.12.2010
(73) Patentinhaber: H.p. Braem Ag, 9402 Mörschwil (CH)
(72) Erfinder: BRAEM, Hans, CH-9402 Mörschwil (CH)
(74) Vertreter: Römpler, Aldo
(86) Internationale Anmeldenummer: PCT/CH2008/000164
(87) Internationale Veröffentlichungsnummer: WO 2009/124406

(56) Entgegenhaltungen:
- WO-A-2008/011225
- US-A- 3 528 410
- US-A- 3 528 425

## Beschreibung

Die vorliegende Erfindung betrifft ein chirurgisches Hilfsmittel für die Ophthalmologie.

Der menschliche Augapfel umfasst einen von einer Aussenhaut umschlossenen Augeninnenraum. Die Aussenhaut ist mehrschichtig, sie wird von der Hornhaut, der Sklera und der Lederhaut gebildet. Der Augeninnenraum unterteilt sich in eine vordere Augenkammer, eine hintere Augenkammer und einen Glaskörperraum, in dem der Glaskörper angeordnet ist. Zwischen der vorderen Augenkammer und dem Glaskörper ist die Augenlinse angeordnet. Der Glaskörper ist von der Netzhaut umgeben, die über den Sehnerv mit dem Gehirn verbunden ist. Die Hornhaut bildet den vorderen transparenten Bereich der Aussenhaut und bildet zusammen mit der in der Augenkammer befindlichen transparenten Flüssigkeit, die überwiegend aus Wasser besteht, der Linse sowie dem Glaskörper ein optisches Abbildungssystem. In dem Augeninnenraum herrscht ein Augendruck oder Innendruck, der grösser ist als der Aussendruck oder Atmosphärendruck.

In der Ophthalmologie sind verschiedene chirurgische Eingriffe bekannt, bei denen mit operativen Instrumenten in das Augeninnere eingegriffen wird. Bei diesen intraokularen invasiven Eingriffen wird wenigstens eine Öffnung im Auge in dessen Aussenhaut erzeugt, durch die ein Instrument in den Augeninnenraum eingeführt wird. Die Vitrektomie ist ein Eingriff oder auch der Teilschritt einer Augenoperation, bei dem gezielt Teile des Glaskörpers chirurgisch entfernt werden. Dabei ist während des Eingriffs ein Druckabfall im Augapfel weitgehend zu vermeiden. Eine Vitrektomie wird häufig im Rahmen einer diabetischen Retinopathie, bei Netzhautablösung oder bei Glaskörperblutungen durchgeführt. Diese können zur partiellen oder vollständigen Erblindung führen.

Bei derartigen Eingriffen erfolgt der Zugang zum Glaskörper durch die Wand, beziehungsweise durch die Aussenhaut des Augapfels. Dabei werden bisher zwei, meistens jedoch drei Schnitte ausgeführt. Einen für das chirurgische Instrument, zum Beispiel ein Vitrektom, eine spezielle Schere, ein Greifer oder ein Häkchen. Damit können der Glaskörper und eventuell an einer Erkrankung beteiligte, weitere Gewebe entfernt werden. Da der Glaskörper ein Gallertgewebe ist, dessen Fasern untereinander kleben, ist ein einfaches Absaugen nicht möglich. Einen weiteren Schnitt wird für die Infusion angelegt, durch die der Augendruck aufrechterhalten wird. Dazu kann der Glaskörperraum zum Beispiel mit einem Irrigationsmedium wie Gas, Silikonöl oder Perfluorcarbonen gefüllt werden. Der meistens unablässige dritte Schnitt dient dem Einführen einer Lichtquelle.

Es ist bekannt, den jeweiligen Bereich der Schnitte in der Aussenhaut des Augapfels durch eine Hülse zu schützen. Dies insbesondere dort, wo das chirurgische Instrument eingeführt wird, denn das Instrument muss während des Eingriffs bewegt werden. Es kann auch vorkommen, dass das Instrument Schwingungen ausführt oder dass nacheinander verschiedene Instrumente zum Einsatz kommen. Die Hülse soll mit ihrer Aussenwand im Schnitt der Aussenhaut des Auges feststecken. Wie sich in der Praxis gezeigt hat, kann es aber immer wieder vorkommen, dass sich die Hülse während des chirurgischen Eingriffs aus der ihr zugedachten Position löst. Oft ist es dabei zunächst so, dass unklar ist, wo die Hülse geblieben ist. Wenn sie nicht am Instrument steckt, dann kann sie auf den Boden gefallen oder, was weniger gut ist, ins Innere des Auges gedrückt worden und eventuell zwischen die Aussenhaut und den Glaskörper gerutscht sein. Die Hülse muss also mühsam gesucht und gefunden werden. Hinzu kommt, dass nach dem Abfallen der Hülse der Schnitt oft kaum mehr zu lokalisieren ist. Diese sehr leicht vorkommenden Zwischenfälle führen in jedem Fall zu einer unnötigen und unangenehmen Verlängerung des Eingriffs und können auch zu Komplikationen führen. Eine Hülse der vorgehend beschriebenen Art ist in WO 2008/011225 erwähnt, wobei auf das vorliegende Problem nicht eingegangen wird. Mehr ist US 3 528 425 zu entnehmen. Der in den Augapfel zu ragen bestimmte Teil der Hülse weist eine im Querschnitt kreisrunde, keilförmige Querschnittserweiterung auf. Beim Einführen in den Augapfel weitet dieser im Querschnitt kreisrunde Keil den Einschnitt in der Aussenhaut des Auges auf. Eine wirksame Verankerung der Hülse ist deshalb nicht gegeben.

Auf der Grundlage dieser Erkenntnisse setzt sich die Erfindung die Aufgabe, ein chirurgisches Hilfsmittel für die Ophthalmologie zu schaffen, durch das die vorgenannten Probleme und Komplikationen vermieden werden können.

Das erfindungsgemässe Hilfsmittel entspricht den kennzeichnenden Merkmalen des Patentanspruchs 1. Weitere vorteilhafte Ausbildungen des Erfindungsgedankens sind aus den abhängigen Patentansprüchen ersichtlich. Dieses Hilfsmittel kann während des Eingriffs nicht mehr verloren gehen. Der chirurgische Eingriff geht nicht nur schneller und problemloser, es sinkt auch die Gefahr, dass es zu Komplikationen bezüglich des Aufrechterhaltens des Augendruckes kommen kann.

Nachfolgend werden bevorzugte Ausführungsbeispiele der Erfindung anhand der Zeichnung näher beschrieben.
- Fig. 1: zeigt eine schematische Ansicht des Augapfels mit dem daran angesetzten Hilfsmittel;
- Fig. 2 - 5: zeigen verschiedene Ansichten des Hilfsmittels nach Fig. 1.

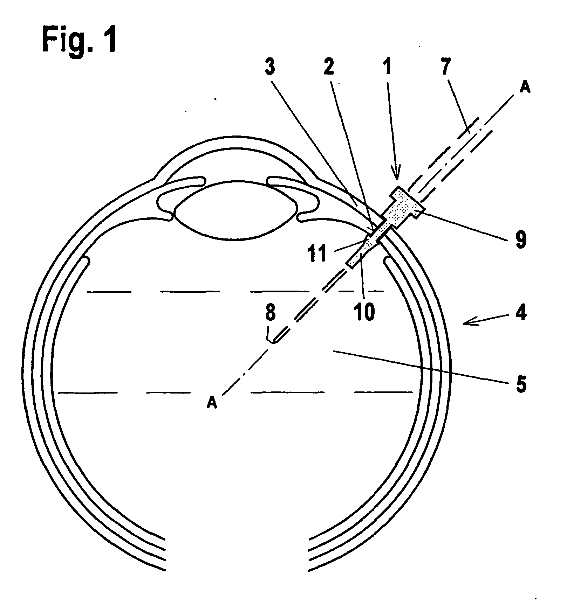

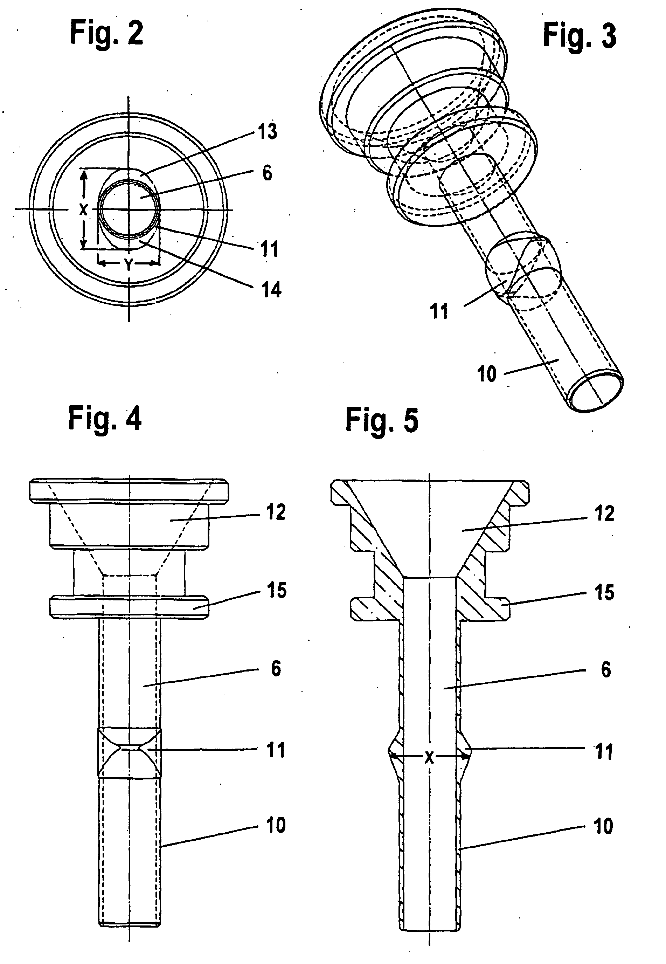

Das hülsenartige Hilfsmittel 1 wird während des chirurgischen Eingriffs, vornehmlich während einer Vitrektomie, in einen Schnitt 2 durch die Aussenhaut 3 des Augapfels 4 eingesetzt, wie dies als Beispiel in Fig. 1 dargestellt ist. Es ragt dabei in diesem Fall bis in den Glaskörper 5 hinein. Durch einen kanalartigen, hohlen Innenraum 6 des Hilfsmittels 1 hindurch, kann beispielsweise ein chirurgisches Instrument 7, das in Fig. 1 nur gestrichelt angedeutet ist, so weit eingeführt werden, dass seine Spitze, beziehungsweise sein distales Ende 8, ins Augeninnere ragt, zum Beispiel in den Glaskörper 5. Der Eintrittswinkel hängt im übrigen von der jeweiligen Operation ab und kann sich während dieser verändern. Derjenige Teil 9 des Hillfsmittels 1 an den das chirurgische Instrument 7 anzusetzen ist, liegt während des Eingriffs an der Aussenseite des Augapfels 4. Der in das Augeninnere, namentlich in den Glaskörper 5 zu ragen bestimmte Teil 10 des Hilfsmittels 1 ist mit mindestens einer Querschnittserweiterung 11 versehen. Der genaue Aufbau des Hilfsmittels 1 geht aus den Fig. 2 - 5 hervor. Das distale Ende 8 des chirurgischen Instruments 7 kann, dank der hier als trichterförmige Aufnahme ausgebildeten proximalen Öffnung 12, gut am Hilfsmittel 1 angesetzt und durch dessen Innenraum 6 hindurch geführt werden, bis sie ins Augeninnere ragt.

Die Querschnittserweiterung 11 ist nicht rotationssymmetrisch sondern länglich ausgebildet, das heisst, dass quer zur Längsachse A - A des Hilfsmittels 1 betrachtet, deren Länge X grösser als die Breite Y ist. Im vorliegenden Beispiel ist die Querschnittserweiterung 11 oval. Sie weist zwei einander gegenüberliegende Auskragungen 13 und 14 auf, die aus dem röhrenförmigen, in den Augapfel 4 zu steckenden Teil 10 des Hilfsmittels 1 ragen. Die Breite Y entspricht hier somit dem Durchmesser des besagten Teils 10. Genau betrachtet handelt es sich in dieser Ausführung also um zwei, einander gegenüberliegenden Querschnittserweiterungen, beziehungsweise Auskragungen 13 und 14. In deren Querschnitt nach Fig. 2 betrachtet, können diese Auskragungen 13 und 14 keilförmig sein, wobei die Keilspitze zum Augeninneren zeigt. Dadurch kann der Teil 10 des Hilfsmittel 1 gut in den Schnitt 2 gesteckt werden und ist dann dort sicher hinter der Aussenhaut 3 verankert.

Zum Einsetzen des Hilfsmittels 1 soll dieses so ausgerichtet werden, dass die Länge X der Querschnittserweiterung 11, beziehungsweise der Auskragungen 13 und 14, in der Ausrichtung des Schnitts 2 der Aussenhaut 3 des Augapfels 4 liegt. Danach kann das Hilfsmittel 1 um seine Längsachse A - A in eine Arretierposition rotiert werden, bei der die Querschnittserweiterung 11 quer zum Schnitt 2 liegt. Beim späteren Herausziehen soll das Hilfsmittel 1 wiederum so weit gedreht werden, bis die Länge X der Querschnittserweiterung 11 in der Ausrichtung des Schnitts 2 liegt.

Um die Rotationslage des Hilfsmittels 1 erkennen zu können, ist es von Vorteil, wenn an dem an der Aussenseite des Augapfels 4 zu liegen bestimmten Teil 9 des Hilfsmittels 1 eine Markierung oder eine Ausformung vorhanden ist, welche die Rotationslage des Hilfsmittels 1 kenntlich macht. Auf diese Weise ist es für den Chirurgen einfach, die Ausrichtung der Querschnittserweiterung 11 festzustellen und das Hilfsmittel 1 schonend aus dem Schnitt 2 zu entfernen, ohne weitere Verletzung der Aussenhaut 3. Um das Hilfsmittel 1 sicher zu positionieren und um zu verhindern, dass es in das Innere des Augapfels 4 gedrückt werden kann, ist eine beispielsweise ringförmige Ausformung 15 möglich.

Die gezeichnete und vorgehend beschriebene Ausführung wird bevorzugt. Alternativ könnte das Hilfsmittel 1 anstelle der Querschnittserweiterung 11 aber auch eine Querschnittsverjüngung aufweisen, die im Bereich des Durchtritts durch die Aussenhaut 3 zu liegen käme. Das heisst, der in das Innere des Augapfels 4 ragende Teil 10 des Hilfsmittels 1 hätte durchgehend einen grösseren Querschnitt als die vorgenannte Querschnittsverjüngung. Ebenso sei ausdrücklich festgehalten, dass die Querschnittserweiterung 11 auch eine von der insbesondere in Fig. 2 dargestellten, ovalen Form abweichende Geometrie aufweisen kann. Dies könnte eine kreisrunde Form oder auch eine beliebige Querschnittserweiterung 11 sein, theoretisch sogar eine mit nur einseitiger Auskragung 13 oder 14.

Um ein allfälliges Heraustreten eines Irrigationsmediums nach oben zu verhindern, kann das Hilfsmittel 1 an seinem aus dem Augapfel 4 herauszuragen bestimmten, proximalen Ende eine vorzugsweise gummielastische Abdichtung aufweisen, beispielsweise aus Silikon. Das Hilfsmittel 1, beziehungsweise sein hülsenartiger Grundkörper, kann vorteilhaft aus Titan bestehen.

## Patentansprüche

1. Chirurgisches Hilfsmittel für die Ophthalmologie, mit einer durch eine Aussenhaut (3) eines Augapfels (4) zu stecken bestimmten, einen durchgehenden, hohlen Innenraum (6) aufweisenden Hülse, durch die ein chirurgisches Instrument (7) einführbar ist, wobei ein in den Augapfel (4) zu ragen bestimmter Teil (10) des Hilfsmittels (1) an dessen Aussenumfang mindestens eine Querschnittserweiterung (11) aufweist, **dadurch gekennzeichnet, dass** die Querschnittserweiterung (11), quer zur Längsachse (A - A) des Hilfsmittels (1) betrachtet, eine Länge (X) aufweist, die grösser als deren Breite (Y) ist, mit dem Zweck, das Hilfsmittel (1) an der Aussenhaut (3) verankern zu können.

2. Chirurgisches Hilfsmittel nach Anspruch 1, **dadurch gekennzeichnet, dass** deren in den Augapfel (4) zu ragen bestimmter Teil (10) mindestens eine Auskragung (13, 14) aufweist.

3. Chirurgisches Hilfsmittel nach Anspruch 2, **dadurch gekennzeichnet, dass** deren in den Augapfel (4) zu ragen bestimmter Teil (10) mindestens zwei einander gegenüberliegende Auskragungen (13, 14) aufweist.

4. Chirurgisches Hilfsmittel nach einem der Ansprüche 1 - 3, **dadurch gekennzeichnet, dass** die Querschnittserweiterung (11) eine ovale Form aufweist.

5. Chirurgisches Hilfsmittel nach einem der Ansprüche 1 - 4, **dadurch gekennzeichnet, dass** die Querschnittserweiterung (11) oder diese Querschnittserweiterung (11) bildende Auskragungen (13, 14) keilförmig ausgebildet ist/sind, wobei die Keilspitze in Richtung des in den Augapfel (4) zu richtenden, distalen Ende des Hilfsmittels (1) zeigt.

6. Chirurgisches Hilfsmittel nach einem der Ansprüche 1 - 5, **dadurch gekennzeichnet, dass** die zum hohlen Innenraum (6) führende Öffnung (12) am aus dem Augapfel (4) herauszuragen bestimmten, proximalen Ende des Hilfsmittels (1) als trichterförmige Aufnahme ausgebildet ist, mit dem Zweck, ein distales Ende (8) des chirurgischen Instrumentes (7) in den Innenraum (6) zu lenken.

7. Chirurgisches Hilfsmittel nach einem der Ansprüche 1 - 6, **dadurch gekennzeichnet, dass** die zum hohlen Innenraum (6) führende Öffnung (12) am aus dem Augapfel (4) herauszuragen bestimmten, proximalen Ende des Hilfsmittels (1) mit einer Abdichtung versehen ist, mit dem Zweck, ein Heraustreten eines Irrigationsmediums zu verhindern.

8. Chirurgisches Hilfsmittel nach einem der Ansprüche 1 - 7, **dadurch gekennzeichnet, dass** an dem an der Aussenseite des Augapfels (4) zu liegen bestimmten Teil (9) des Hilfsmittels (1) eine Markierung oder eine Ausformung vorhanden ist, mit dem Zweck, die Rotationslage des Hilfsmittels (1) kenntlich zu machen.

## Claims

1. A surgical aid for ophthalmology, having a sleeve which in turn has a continuous hollow interior space (6) and is intended for insertion through an outer member (3) of an eyeball (4), a surgical instrument (7) being insertable through this sleeve, such that a portion (10) of the aid (1) intended for protruding into the eyeball (4) has at least one enlarged cross section (11) on its outer circumference,
**characterized in that**
the enlarged cross section (11), when observed across the longitudinal axis (A-A) of the aid (1), has a length (X) greater than its width (Y), with the purpose of being able to anchor the aid (1) on the outer membrane (3).

2. The surgical aid for ophthalmology according to Claim 1,
**characterized in that**
its portion (10) intended for protruding into the eyeball (4) has at least one cantilever arm (13, 14).

3. The surgical aid according to Claim 2,
**characterized in that**
its portion (10) intended for protruding into the eyeball (4) has at least two opposing cantilever arms (13, 14).

4. The surgical aid according to any one of Claims 1 to 3,
**characterized in that**
the enlarged cross section (11) has an oval shape.

5. The surgical aid according to any one of Claims 1 to 4,
**characterized in that**
the enlarged cross section (11) or the cantilever arms (13, 14) forming this enlarged cross section (11) is/are designed with a wedge shaped, such that the tip of the wedge points in the direction of the distal end of the aid (1) directed into the eyeball (4).

6. The surgical aid according to any one of Claims 1 to 5,
**characterized in that**
the opening (12) on the proximal end of the aid (1) intended for protruding out of the eyeball (4), said opening leading to the hollow interior space (6), is designed as a funnel-shaped receptacle with the purpose of deflecting the distal end (8) of the surgical instrument (7) into the interior space (6).

7. The surgical aid according to any one of Claims 1 to 6,
**characterized in that**
the opening (12) on the proximal end of the aid (1) intended for protruding out of the eyeball (4), said opening leading into the hollow interior space (6), is provided with a seal with the purpose of preventing an irrigation medium from escaping.

8. The surgical aid according to any one of Claims 1 to 7,
**characterized in that**
a mark or a recess is provided on the portion (9) of the aid (1) intended to lie on the outside of the eyeball (4), with the purpose of identifying the rotational position of the aid.

## Revendications

1. Accessoire chirurgical à usage ophtalmologique, comprenant une douille présentant un intérieur (6) creux traversant et destinée à être insérée à travers une enveloppe extérieure (3) d'un globe oculaire (4), et par laquelle un instrument chirurgical (7) peut être introduit, dans lequel une partie (10) de l'accessoire (1), destinée à faire saillie dans le globe oculaire (4), présente sur la circonférence extérieure de celle-ci au moins un élargissement de section transversale (11), **caractérisé en ce que** l'élargissement de section transversale (11), vu transversalement à l'axe longitudinal (A-A) de l'accessoire (1), présente une longueur (X) supérieure à la largeur (Y) de celui-ci, en vue de permettre l'ancrage de l'accessoire (1) dans l'enveloppe extérieure (3).

2. Accessoire chirurgical selon la revendication 1, **caractérisé en ce que** sa partie (10) destinée à faire saillie dans le globe oculaire (4) présente au moins une saillie (13, 14).

3. Accessoire chirurgical selon la revendication 2, **caractérisé en ce que** sa partie (10) destinée à faire saillie dans le globe oculaire (4) présente au moins deux saillies (13, 14) opposées l'une à l'autre.

4. Accessoire chirurgical selon l'une quelconque des revendications 1 à 3, **caractérisé en ce que** l'élargissement de section transversale (11) présente une forme ovale.

5. Accessoire chirurgical selon l'une quelconque des revendications 1 à 4, **caractérisé en ce que** l'élargissement de section transversale (11) ou les saillies (13, 14) formant ledit élargissement de section transversale (11) est/sont réalisé(es) en forme de clavette, dans lequel la pointe de clavette est tournée en direction de l'extrémité distale de l'accessoire (1) qui est à diriger dans le globe oculaire (4).

6. Accessoire chirurgical selon l'une quelconque des revendications 1 à 5, **caractérisé en ce que** l'orifice (12) donnant sur l'intérieur creux (6) est réalisé comme un logement en forme d'entonnoir au niveau de l'extrémité proximale de l'accessoire (1) qui est destinée à faire saillie hors du globe oculaire (4), en vue de diriger une extrémité distale (8) de l'instrument chirurgical (7) vers l'intérieur (6).

7. Accessoire chirurgical selon l'une quelconque des revendications 1 à 6, **caractérisé en ce que** l'orifice (12) donnant sur l'intérieur creux (6) est munie d'un joint d'étanchéité au niveau de l'extrémité proximale de l'accessoire (1) qui est destinée à faire saillie hors du globe oculaire (4), en vue d'empêcher des fuites d'un milieu d'irrigation.

8. Accessoire chirurgical selon l'une quelconque des revendications 1 à 7, **caractérisé en ce que** sur la partie (9) de l'accessoire (1), destinée à être placée sur le côté extérieur du globe oculaire (4), il existe un marquage ou une formation en vue d'identifier la position de rotation de l'accessoire (1).
